# EUROPEAN PATENT APPLICATION

(11) **EP 4 722 180 A1**
(43) Date of publication of application: **08.04.2026**
(21) Application number: 25204564.6
(22) Date of filing: 25.09.2025
(51) Int. Cl.: C04B 35/486, A61K 6/818, C04B 35/488, C04B 35/64, C04B 41/45, A61C 13/083

(54) **ZIRCONIA MILL BLANK**

(30) Priority: 27.09.2024 JP 2024169642; 29.05.2025 JP 2025090195; 29.05.2025 JP 2025090214
(71) Applicant: Shofu Inc., Kyoto-shi, Kyoto 605-0983 (JP)
(72) Inventor: NONAKA, Kazumichi, Kyoto-shi, Kyoto, 605-0983 (JP); ITO, Shuma, Kyoto-shi, Kyoto, 605-0983 (JP)
(74) Representative: Eisenführ Speiser

(57) **Abstract**

To provide a zirconia mill blank for dental cutting and machining that can reduce the number of type of the zirconia mill blank for dental cutting and machining that dental laboratories and similar facilities must stock in order to obtain a zirconia sintered body with excellent translucency. To provide a zirconia mill blank for dental cutting and machining for obtaining zirconia sintered bodies having different optical properties by different sintering temperatures, wherein the different sintering temperatures are selected from at least a range of 1500 °C to 1600 °C and a range of 1400 °C to 1500 °C.

## Description

### BACKGROUND OF THE INVENTION

### Field of the Invention

The present invention relates to a zirconia mill blank for dental cutting and machining.

### Description of the Related Art

In recent years, techniques to prepare a prosthesis device by cutting and machining which uses the dental CAD/CAM system have been spread rapidly and therefore it has been becoming possible to easily prepare prosthesis devices by cutting and machining mill blanks which are made of ceramic materials such as a zirconia, an alumina and a lithium disilicate glass or resin materials such as an acrylic resin and a hybrid resin.

In particular, the zirconia has been clinically applied in various cases because of its high strength. On the other hand, the fully sintered zirconia (hereinafter, also referred to as "zirconia fully sintered body") has very high hardness and therefore cannot be cut and machined using a dental CAD/CAM system. Thus, a zirconia which is not perfect sintered but is calcined at a low firing temperature to adjust to a hardness that enables to cut has been used as a zirconia mill blank for dental cutting and machining.

A general zirconia mill blank for dental cutting and machining is manufactured by molding a zirconia powder by press molding or the like and then calcining at 800 to 1200 °C.

The properties of the zirconia mill blank for dental cutting and machining, that is, the properties of the zirconia fully sintered body (perfect sintered body) are influenced by the properties of the used zirconia powder.

Japanese Unexamined Patent Application Publication No. 2022-184793 discloses that a zirconia sintered body having excellent translucency can be obtained by sintering a zirconia pre-sintered body having a specific stabilizer concentration according to a specific sintering schedule.

### SUMMARY OF THE INVENTION

### Technical Problem

In the zirconia sintered bodies having excellent translucency as described in Japanese Unexamined Patent Application Publication No. 2022-184793, although it has been easy to reproduce the shades of natural teeth, it has been difficult to adjust the shades in cases where the abutment tooth is significantly discolored or the translucency of an adjacent tooth is low.

Therefore, it is necessary to use zirconia mill blanks for dental cutting and machining with different translucency depending on the clinical cases.

Thus, dental laboratories and other facilities have been rquired to keep various types of zirconia mill blanks for dental cutting and machining in stock.

An object of the present invention is to provide a zirconia mill blank for dental cutting and machining that can reduce the number of type of the zirconia mill blank for dental cutting and machining that dental laboratories and similar facilities must stock.

### Solution to Problem

The present inventors made a study on a zirconia mill blank for dental cutting and machining that can reduce the number of type of the zirconia mill blank for dental cutting and machining that dental laboratories and similar facilities must stock in order to obtain a zirconia sintered body with excellent translucency. As a result, it has been found that it is possible to obtain sintered bodies having different optical properties by changing the sintering temperature. The details of the present invention are as follows.

The present invention provides, as one embodiment, a zirconia mill blank for dental cutting and machining for obtaining zirconia sintered bodies having different optical properties by different sintering temperatures, wherein the different sintering temperatures are selected from at least a range of 1500 °C to 1600 °C and a range of 1400 °C to 1500 °C.

The present invention provides, as one embodiment, a zirconia mill blank for dental cutting and machining for obtaining zirconia sintered bodies having different optical properties by different sintering temperatures, wherein the different sintering temperatures are in a range of 1400 °C to 1600 °C, and a difference in sintering temperature bewtween the different sintering temperatures is 50 °C to 150°C.

The present invention provides, as one embodiment, a zirconia mill blank for dental cutting and machining for obtaining zirconia sintered bodies having different optical properties by different sintering temperatures, wherein a difference between a total light transmittance of sintered body having a thickness of 1mm produced by sintering at one sintering temperature of the different sintering temperatures for 1 hour and a total light transmittance of sintered body having a thickness of 1mm produced by sintering at another sintering temperature of the different sintering temperatures for 1 hour is 0.5 % pt or more, and a total light transmittance at light of wavelength of 700 nm in the case of sintering at a temperature in a range of 1500 °C to 1600 °C is 37 % or more.

The present invention provides, as one embodiment, zirconia mill blank for dental cutting and machining for obtaining zirconia sintered bodies having different optical properties by different sintering temperatures, wherein a crystal grain size of sintered body in the case of producing by sintering at one sintering temperature of the different sintering temperatures is 2.0 times or more of a crystal grain size of sintered body in the case of producing by sintering at another sintering temperature of the different sintering temperatures.

In the present invention, the crystal grain size of sintered body produced by sintering at 1550 °C for 1 hour may be 3.0 µm or more.

In the present invention, the stabilizer may be yttria.

In the present invention, it may further contain a coloring element.

In the present invention, a difference between a total light transmittance at light of wavelength of 700 nm of sintered body having a thickness of 1mm in the case of producing by sintering at 1550 °C for 1 hour and a total light transmittance at light of wavelength of 700 nm of sintered body having a thickness of 1mm in the case of producing by sintering at 1450 °C for 1 hour may be 0.5% pt or more.

### Advantageous Effects of Invention

The zirconia mill blank for dental cutting and machining of the present invention can reduce the number of type of the zirconia mill blank for dental cutting and machining that dental laboratories and similar facilities must stock in order to obtain a zirconia sintered body with excellent translucency.

### DETAILED DESCRIPTION OF THE PREFERRED EMBODIMENTS

In one aspect, it is possible to understand the zirconia mill blank for dental cutting and machining of the present invention as a zirconia mill blank for dental cutting and machining for obtaining zirconia sintered bodies having different optical properties by different sintering temperatures, wherein the different sintering temperatures are selected from at least a range of 1500 °C to 1600 °C and a range of 1400 °C to 1500 °C.

In one aspect, it is possible to understand the zirconia mill blank for dental cutting and machining of the present invention as a zirconia mill blank for dental cutting and machining for obtaining zirconia sintered bodies having different optical properties by different sintering temperatures, wherein the different sintering temperatures are in a range of 1400 °C to 1600 °C, and a difference in sintering temperature bewtween the different sintering temperatures is 50 °C to 150°C.

In one aspect, it is possible to understand the zirconia mill blank for dental cutting and machining of the present invention as A zirconia mill blank for dental cutting and machining for obtaining zirconia sintered bodies having different optical properties by different sintering temperatures, wherein a difference between a total light transmittance of sintered body having a thickness of 1mm produced by sintering at one sintering temperature of the different sintering temperatures for 1 hour and a total light transmittance of sintered body having a thickness of 1mm produced by sintering at another sintering temperature of the different sintering temperatures for 1 hour is 0.5 % pt or more, and a total light transmittance at light of wavelength of 700 nm in the case of sintering at a temperature in a range of 1500 °C to 1600 °C is 37 % or more.

In one aspect, it is possible to understand the zirconia mill blank for dental cutting and machining of the present invention as a zirconia mill blank for dental cutting and machining for obtaining zirconia sintered bodies having different optical properties by different sintering temperatures, wherein a difference between a total light transmittance of sintered body having a thickness of 1mm produced by sintering at one sintering temperature of the different sintering temperatures for 1 hour and a total light transmittance of sintered body having a thickness of 1mm produced by sintering at another sintering temperature of the different sintering temperatures for 1 hour is 0.5% pt or more, and a total light transmittance at light of wavelength of 700 nm in a case of sintering at a temperature in a range of 1500 °C to 1600 °C is 37 % or more

In one aspect, it is possible to understand the zirconia mill blank for dental cutting and machining of the present invention as a zirconia mill blank for dental cutting and machining for obtaining zirconia sintered bodies having different optical properties by different sintering temperatures, wherein a crystal grain size of sintered body in the case of producing by sintering at the first sintering temperature is 2.0 times or more of a crystal grain size of sintered body in the case of producing by sintering at the second sintering temperature.

In one aspect, it is possible to understand the zirconia mill blank for dental cutting and machining of the present invention as a zirconia mill blank for dental cutting and machining for obtaining zirconia sintered bodies having different optical properties by different sintering temperatures, wherein a crystal grain size of sintered body in the case of producing by sintering at one sintering temperature of the different sintering temperatures is 2.0 times or more of a crystal grain size of sintered body in the case of producing by sintering at another sintering temperature of the different sintering temperatures.

In one aspect, it is possible to understand the zirconia mill blank for dental cutting and machining of the present invention as a zirconia mill blank for dental cutting and machining for obtaining zirconia sintered bodies having different optical properties by different sintering temperatures.

Hereinafter, specific embodiments of the requirments of the present invention will be described in detail.

The zirconia mill blank for dental cutting and machining of the present invention is a zirconia mill blank for dental cutting and machining for producing a prosthesis device by the cutting and machining. This zirconia mill blank for dental cutting and machining is a semi-fired zirconia mill blank for dental cutting and machining that includes a semi-fired body (calcined body/pre-sintered body) of ceramic particles and can be manufactured using known zirconia raw material powder.

The zirconia mill blank for dental cutting and machining of the present invention may contain, for example, a zirconium oxide and a stabilizer consisting of an oxide (hereinafter also referred to simply as "stabilizer" in the present specification). In the present invention, the content of the stabilizer may be, for example, 2.0 mol % or more and 7.0 mol % or less in terms of oxide, or 5.8 mol % or more and and 7.0 mol % or less in terms of oxide. In the present invention, the content of the stabilizer refers to a value expressed by mol % of the molar ratio of (stabilizer content) / (the content of all inorganic oxide contained in the semi-fired body of the ceramic particles).

In the present invention, the amount of stabilizer can be determined by elemental analysis of the sample. There is no particular limitation on the elemental analysis method, but for example, X-ray fluorescence analysis can be used.

The zirconia mill blank for dental cutting and machining of the present invention may contain substantially no alumina.

In the present invention, the phrase "substantially no alumina" means that it is not contain alumina in an amount that affects the properties of the zirconia mill blank for dental cutting and machining or the zirconia sintered body obtained by sintering the zirconia mill blank for dental cutting and machining, and specifically means that an alumina content is 500 ppm by weight or less of the zirconia mill blank for dental cutting and machining. The alumina content may be 300 ppm or less, and may be 200 ppm or less. Depending on the raw material of zirconia, there is a case where a trace amount of alumina may be unavoidably contained even when alumina is not intentionally added, but in such cases, the alumina content is generally 500 ppm or less. The alumina content can be determined by various elemental analyses. There is no particular limitation on the method of elemental analysis, but for example, fluorescent X-ray analysis can be used. At this time, depending on measurement condition and analysis condition, there is a case where alumina may be detected even in a sample that does not actually contain alumina, but in such cases the alumina content is generally 500 ppm or less.

The stabilizer contained in the zirconia mill blank for dental cutting and machining of the present invention is not particularly limited, but yttria may be used because it is easily available and is widely used in dental zirconia. For other stabilizers, ytterbium oxide, in addition to erbium oxide, neodymium oxide, praseodymium oxide and terbium oxide which are also effective as coloring agents may be used. In the present invention, the stabilizer may be one or more selected from yttrium oxide, magnesium oxide, calcium oxide, scandium oxide, gallium oxide, indium oxide and lanthanide oxides.

The zirconia mill blank for dental cutting and machining of the present invention may contain a coloring element. Specific examples thereof include iron for imparting a yellow color and erbium for imparting a red color. In addition, there is no problem at all even if cobalt, manganese and chromium for adjusting a shade in addition to these coloring element used together. In the present invention, the tooth color can be easily colored by including the coloring element.

The stabilizer contained in the zirconia mill blank for dental cutting and machining of the present invention may exist as a solid solution within the zirconia crystal or may exist as a stabilization element compound without being solid solved as a stabilizer. Specifically, the stabilization element compound may be a compound selected from oxides, halide compounds, nitrates, sulfates and/or organic acid salts. Furthermore, the coloring element contained in the zirconia mill blank for dental cutting and machining in the present invention may exist in zirconia crystals and may exist as a coloring element compound separate from the zirconia crystals. Specific examples in the case of presenting in the zirconia crystal include the case where the coloring element is present at the zirconium site in the zirconia crystal and the case where the coloring element is present at the interstitial site in the zirconia crystal. The coloring element compound separate from the zirconia crystals may specifically be a compound consisting of any one of oxides, halogen compounds, nitrates, sulfates and organic acid salts of the coloring elements.

Specific examples of the water-soluble stabilization element compound include yttrium chloride, yttrium nitrate, yttrium acetate, yttrium carboxylate, yttrium sulfate, yttrium carbonate, ytterbium chloride, ytterbium nitrate, ytterbium acetate, ytterbium carboxylate, ytterbium sulfate, ytterbium carbonate, erbium chloride, erbium nitrate, erbium acetate, erbium carboxylate, erbium sulfate, erbium carbonate, neodymium chloride, neodymium nitrate, neodymium acetate, neodymium carboxylate, neodymium sulfate, neodymium carbonate, praseodymium chloride, praseodymium nitrate, praseodymium acetate, praseodymium carboxylate, praseodymium sulfate, praseodymium carbonate, terbium chloride, terbium nitrate, terbium acetate, terbium carboxylate, terbium sulfate and terbium carbonate. Specific examples of the coloring element compound include iron chloride, iron nitrate, iron acetate, nickel chloride, nickel nitrate and nickel acetate.

Among water-soluble stabilization element compound and/or water-soluble coloring element compound, an organic acid salt is particularly preferable from the viewpoint of low decomposition temperature and suppressing the contamination of the firing furnace and the like. Specific examples include yttrium acetate and yttrium carbonate. The organic acid salt decomposes at lower temperature in comparison with the inorganic salt such as halogen compound, nitrate and sulfate. When the decomposition temperature is high, it may be difficult to impart sufficient translucency and strength to the zirconia fully sintered body because a pore remains in sintering process.

The content of the water-soluble stabilization element compound may be, for example, 0.1 to 3.5 mol %, or 0.5 to 3.0 mol %. In the present invention, the content of the water-soluble stabilization element compound refers to a value expressed by mol % of the molar ratio of (water-soluble stabilization element compound content) / (the content of all inorganic oxide contained in the zirconia mill blank for dental cutting and machining).

When the content of the water-soluble stabilization element compound is less than 0.1 mol %, there is a case where it is impossible to impart sufficient translucency to the zirconia mill blank for dental cutting and machining. On the other hand, when the content exceeds 3.5 mol %, although the translucency of the zirconia fully sintered body is improved, there is a case where it is difficult to impart sufficient strength. In the present invention, the water-soluble stabilization element compound may contain yttria. When the water-soluble stabilization element compound contains yttria, it is possible to further improve the translucency.

The zirconia mill blank for dental cutting and machining in the present invention may contain additives other than the stabilizer and the coloring agent. Specific examples of additives include compounds of gallium, lanthanum, niobium and tantalum. By addition of these additives, it is possible to control sintering behavior, to reduce translucency, and to improve fracture toughness. These additives may be solid solved or may not be solid solved in zirconia.

The zirconia mill blank for dental cutting and machining of the present invention may contain hafnium. The content of hafnium may be, for example, 2 % by weight or less.

As the zirconia powder used for manufacturing the zirconia mill blank for dental cutting and machining of the present invention, any zirconia powder manufactured from a known zirconia powder may be used without any limitations. Specifically, the zirconia powder used for manufacturing the zirconia mill blank for dental cutting and machining of the present invention may be produced by a hydrolysis method. More specifically, the zirconia powder is produced by a method which comprises heating a solution in which a zirconium salt and a yttrium compound are mixed and dissolved to cause a hydrolysis reaction, drying and firing the generated sol to prepare a zirconia powder, and pulverizing and granulating the powder. Further, by mixing an aluminum compound before this pulverization step, an alumina-containing zirconia powder is prepared.

It is preferable that the primary particle diameter of the zirconia powder used for manufacturing the zirconia mill blank for dental cutting and machining in the present invention is 10 to 500 nm. When the primary particle diameter is less than 10 nm, there is a tendency that it is difficult to impart sufficient strength to the zirconia sintered body. On the other hand, when the primary particle diameter is more than 500 nm, there is a tendency that it is difficult to impart sufficient strength to the zirconia sintered body.

The relative density of the zirconia sintered body prepared by firing the zirconia mill blank for dental cutting and machining of the present invention at 1550 °C may be 98% or more of the theoretical density. The relative density is determined by the measured density/the theoretical density. When the relative density is less than 98%, there is a tendency that the strength and translucency are lowered.

It is preferable that a crystal phase of the zirconia mill blank for dental cutting and machining of the present invention is tetragonal and/or cubic. When the crystal phase is monoclinic phase, it is not preferable because sufficient translucency may be not imparted after fully sintering of zirconia.

The zirconia mill blank for dental cutting and machining of the present invention can provide a zirconia sintered bodies having different optical properties by different sintering temperatures.

The sintering temperature of the zirconia mill blank for dental cutting and machining of the present invention is not particularly limited, and may be selected, for example, from a temperature range of 1400 °C to 1650 °C, from a temperature range of 1400 °C to 1600°C, and from a temperature range of 1450 °C to 1600°C. In other words, the "different sintering temperatures" of the present invention are not particularly limited, and may be, for example, two or more temperatures selected from a temperature range of 1400 °C to 1650 °C, from a temperature range of 1400 °C to 1600 °C, and from a temperature range of 1450 °C to 1600 °C.

Specifically, the "different sintering temperatures" of the present invention may be temperatures in two or more different temperature ranges selected from, for example, a temperature range of 1400 °C to 1450 °C, a temperature range of 1450 °C to 1500 °C, a temperature range of 1500 °C to 1550 °C, a temperature range of 1550 °C to 1600 °C, and a temperature range of 1600 °C to 1650 °C. Furthermore, the "different sintering temperatures" of the present invention may be temperatures in two or more different temperature ranges selected from, for example, a temperature range of 1400 °C to 1500 °C, a temperature range of 1450 °C to 1550 °C, a temperature range of 1500 °C to 1600 °C, and a temperature range of 1550 °C to 1650 °C.

In the present invention, the temperature ranges from which the "different sintering temperatures" are selected may be temperature ranges that do not overlap with each other except for their endpoints.

More specifically, when the "different sintering temperatures" of the present invention are two temperatures, they may be selected from, for example, a temperature range of 1400 °C to 1500 °C and a temperature range of 1500 °C to 1600 °C, and from a temperature range of 1450 °C to 1550 °C and a temperature range of 1550 °C to 1650°C, or from a temperature range of 1400 °C to 1500 °C and a temperature range of 1550 °C to 1650 °C. In particular, when the "different sintering temperatures" of the present invention are two temperatures, they may be selected from a temperature range of 1400 °C to 1500 °C and a temperature range of 1500 °C to 1600°C.

The "different sintering temperatures" of the present invention may be two or more different temperatures selected from the same temperature range. In this case, the same temperature range may be, for example, a temperature range of 1400 °C to 1650 °C, and a temperature range of 1450 °C to 1600 °C. Furthermore, the temperature difference between the two or more temperatures may be 50 °C or more, may be in a range of 50 °C to 150 °C, may be in a range of 50 °C to 100 °C, and may be in a range of 100 °C to 150°C.

More specifically, when the "different sintering temperatures" of the present invention are two temperatures, the two temperatures may be selected from a temperature range of, for example, 1400 °C to 1600 °C, and a difference between the sintering temperatures may be 50 °C to 150 °C.

In the present invention, there is no limitation on the method for procuding zirconia sintered bodies having different optical properties based on different sintering temperatures. Specifically, it is possible to add an element that changes the optical properties of the produced zirconia sintered body by sintering at a different sintering temperature. Examples of such element include gallium, indium, titanium, scandium, barium, niobium and tantalum. In order to add these elements to the zirconia mill blank for dental cutting and machining, they may be added by mixing a solution containing these elements into a zirconium oxychloride solution, which is the zirconia raw material, they may be added by mixing a zirconia powder with a compound containing these elements and then granulating, and they may be added by permeating the zirconia mill blank for dental cutting and machining with a solution containing these elements.

The optical properties of the zirconia sintered body are affected by, for example, the crystal grain size of the manufactured zirconia sintered body. In the present invention, "crystal grain size" refers to the diameter of a circle with the same area as the crystal grain (Heywood diameter). There is no limitation on the measuring method of the crystal grain size. For example, it can be measured by observing the zirconia sintered body using SEM and then using commercially available particle image analysis software on the obtained images. In this measurement method, in order to prevent bias in the measured values, the crystal grain size is measured for 50 or more randomly selected crystal grains, and the median diameter is taken as the crystal grain size of the sample.

There is a case where a stabilizer element such as yttrium affects the grain size difference. Generally, yttrium is added in the form of a solution during zirconia powder production. The yttrium added in this manner is present almost uniformly in the zirconia particles. On the other hand, yttrium may be added by mixing a zirconia powder and a yttria powder and may be added by permeating a zirconia calcined body with an yttrium solution. The yttrium added in thes manners exists on the outside of the zirconia particles. This difference in yttrium distribution affects the crystal grain size difference in zirconia sintered bodies, and there is a tendency that the difference in the crystal grain size is large in the case of adding yttrium to the outside of the zirconia particles. In other words, the yttrium added to the outside of the zirconia particles acts not only as a stabilizer but also as a grain size difference adjuster.

The reason for the difference in crystal grain size depending on the adding method of yttrium is unclear. However, there is a possibility that the mobility of crystal grain boundary due to uneven distribution of yttrium affects. The yttrium added to the outside of the zirconia particles solid solves in the zirconia during sintering. At this time, since the yttrium cannot diffuse into the interior of the zirconia particles, the yttrium concentration near the surface of the zirconia particles becomes higher than that of the interior. On the other hand, when yttrium is uniformly added to zirconia particles, the difference in yttria concentration between the surface and the interior of the zirconia particles is relatively small. It is considered that the concentration of impurity ions near the grain boundary strongly affects the mobility of the grain boundary and to generate a difference in crystal grain size.

A specific example of the difference in crystal grain size that affects the optical properties of zirconia sintered body include that the crystal grain size of one of the sintered bodies produced by sintering at different sintering temperatures for one hour is at least 2.0 times the crystal grain size of the other. More specifically, for example, the crystal grain size of a sintered body produced by sintering at 1550 °C for one hour is at least 2.0 times the crystal grain size of a sintered body produced by sintering at 1450 °C for one hour, or the crystal grain size of a sintered body produced by sintering at 1500 °C for one hour is at least 2.0 times the crystal grain size of a sintered body produced by sintering at 1600 °C for one hour.

When the crystal grain size of one of the sintered bodies produced by sintering at different sintering temperatures for 1 hour is less than 2.0 times of a crystal grain size of another of the sintered bodies produced by sintering at different sintering temperatures for 1 hour, the change in translucency, strength and color based on the change in sintering temperature is small, therefore is is impossible to adjust translucency, strength and color by changing the sintering temperature depending on the clinical case. The crystal grain size of one of the sintered bodies produced by sintering at different sintering temperatures for 1 hour may be 2.3 times or more, may be 2.5 times or more, and may be 2.7 times or more of the crystal grain size of another of the sintered bodies produced by sintering at different sintering temperatures for 1 hour. By satisfying such relationship in the crystal grain size the change in translucency, strength and color based on the change in sintering temperature becomes large, and therefore it is possible to adjust translucency, strength and color by changing the sintering temperature depending on the case.

It is preferable that the amount of stabilizer in terms of oxide is 5.8 mol % or more. When the amount of stabilizer in terms of oxide is less than 5.8 mol %, the change in translucency, strength and color based on the change in sintering temperature is small, a problem occurs in that it is not possible to adjust the translucency, the strength and the color by changing the sintering temperature depending on the clinical case. It is preferable that the amount of stabilizer in terms of oxide is 7.0 mol % or less. When the amount of the stabilizer in terms of oxide exceeds 7.0 mol %, a problem occurs in that the translucency and/or the strength decrease. The amount of the stabilizer in terms of oxide may be 6.2 mol % or more and 7.0 mol % or less. By setting in such range, the change in translucency, strength and color based on the change in sintering temperature becomes large, it becomes easier to adjust the translucency, strength and color by changing the sintering temperature depending on the clinical case.

The crystal grain size of one of the sintered bodies produced by sintering at different sintering temperatures may be 2.0 µm or more, may be 3.0 µm or more, and may be 4.0 µm or more. In this case, the change in translucency in the case of changing the sintering temperature is large, and it is possible to incerase the number of clinical case that can be accommodated by adjusting the sintering temperature.

In the present invention, a difference between a total light transmittance at light of wavelength of 700 nm of sintered body having a thickness of 1mm producing from one of the zirconia sintered bodies obtained by sintering at different sintering temperatures and a total light transmittance at light of wavelength of 700 nm of sintered body having a thickness of 1mm producing from the other of the zirconia sintered bodies obtained by sintering at different sintering temperatures may be 0.5 % pt or more. In this case, it is possible to adjust the translucency according to the clinical case by changing the sintering temperature. The difference in total light transmittance may be 2 % pt or more, or may be 3 % pt or more. By achieving such a difference in total light transmittance, it is possible to increase the range for adjusting translucency.

In the present invention, the total light transmittance at 700 nm in the case of sintering at any temperature in a range of 1500 °C to 1600 °C may be 37 % or more, and may be 38 % or more. In this case, it is possible to obtain a zirconia sintered body having excellent translucency.

The manufacturing method of the zirconia mill blank for dental cutting and machining of the present invention is not particularly limited, and any known manufacturing methods can be used without any problem. Specifically, it is preferable to be manufactured by molding a zirconia powder by a press molding. Furthermore, it may be manufactured by a multilayer molding in which zirconia powders having different color tones or compositions are press-molded in multiple stages.

The zirconia mill blank for dental cutting and machining of the present invention is preferably subjected to isostatic pressing by cold isostatic pressing (CIP treatment) after the press molding.

The maximum load pressure of CIP treatment in the present invention is preferably 50 Mpa or more. When the maximum load pressure is less than 50 MPa, there is a case where sufficient translucency and strength may be not imparted to the zirconia sintered body.

The holding time at the maximum load pressure of the CIP treatment of the present invention is not particularly limited, but in general, 0 to 150 seconds is preferable and 0 to 60 seconds is more preferable.

The time period required for the above series of processes is not particularly limited, but is usually preferably 30 seconds to 10 minutes and is more preferably 3 minutes to 7 minutes. When the time is too short, a molding body may be destroyed, and when the time is too long, production efficiency worsens, and therefore these are not preferable.

A calcination temperature of the zirconia mill blank for dental cutting and machining of the present invention is preferably 800 to 1200 °C. When the calcination temperature is less than 800 °C, because Vickers hardness and/or bending strength become too low and therefore there is a tendency that chipping and breakage easily occur in cutting and machining. On the other hand, when the calcination temperature is more than 1200 °C, because Vickers hardness and/or bending strength become too high and therefore there is a tendency that a milling bar of a milling machine is heavily consumed to raise a running cost.

The process of manufacturing a zirconia mill blank for dental cutting and machining in the present invention may include a step of impregnating with an impregnating solution containing metal ion. Specifically, at least one type of impregnating solution is permeated into the zirconia calcined body, and the metal compound is supported in a zirconia pore by drying, degreasing and other processes. By this step, it is possible to impart the desired translucency, color tone, mechanical property and the like to a desired area of the zirconia mill blank for dental cutting and machining. It is preferable to use two or more kinds of impregnating solution. In this case, smooth gradation of translucency and/or shade and/or mechanical property may be imparted to the zirconia mill blank by ionic diffusion between the impregnating solutions. An impregnating solution that does not contain metal ions may also be used for ion diffusion.

At least one of the metal ion contained in the impregnating solution may be a rare earth metal ion. Specific examples of the rare earth metal ion include an yttrium ion. By containing a rare earth metal ion, it is possible to improve the translucency of the zirconia sintered body and to color the zirconia sintered body. The metal ion may be only a rare earth metal ion.

At least one of the metal ion contained in the impregnating solution may be a transition metal ion. Specific transition metal ion is an iron ion. By containing a transition metal ion, it is possible to color a zirconia sintered body. The metal ion may be only a transition metal ion.

At least one of the metal ion contained in impregnating solution may be any one or more of an aluminum ion, a gallium ion and an indium ion. By containing any one or more of aluminum ion, gallium ion and indium ion, it is possible to improve the sinterability of the zirconia sintered body and to improve the translucency of the zirconia sintered body. The metal ion may be only an aluminum ion, a gallium ion and/or an indium ion. The metal ion may be only a rare earth metal ion, a transition metal ion, an aluminum ion, a gallium ion and/or an indium ion.

The metal ion concentration of the impregnating solution in the present invention is not particularly limited, but in the case of an yttrium ion, for example, it is preferably about 2.0 % by weight to 10.0 % by weight, and more preferably about 4.0 % by weight to 10.0 % by weight. When it is 2.0 % by weight or less, there is a case that the amount of supported yttrium is small and sufficient characteristic is not obtained. When it is 10.0 % by weight or more, there is a case that the amount of supported yttrium is large and physical property is adversely affected. Since the optimum amount of supported metal depends on the type of metal and the desired characteristics, the metal ion concentration of the impregnating solution is preferably determined from the desired optimum amount of supported metal and the solubility of the metal ion source in the solvent.

The solvent used in the impregnating solution is not particularly limited, but water is preferable since it is easily available and is easy to handle.

The impregnating solution may be added with an acid or a base, or a pH adjusting agent for the purpose of controlling the pH. The acid or base to be used is not particularly limited, but it is preferable to use an organic acid such as acetic acid, citric acid and the like or aqueous ammonia from the viewpoint of leaving no residue on the porous zirconia molded body.

The impregnating solution may contain a precipitant. Precipitant precipitates a metal compound by heat treatment or other operation after permeation of the impregnating solution, and specific examples include urea and hexamethylenetetramine. Urea is preferable since it is easily available and is easy to handle. This can suppress segregation of a metal compound.

The impregnating solution may contain various additives. Examples of additives include glycols and polymers that adjust the viscosity of the impregnating solution, and chelating agents that increase the stability of a metal ion. Specific examples of the former include ethylene glycol, propylene glycol, polyethylene glycol and the like. Specific examples of the latter include citric acid, ammonium ethylenediamine tetraacetate and the like. The impregnation solution may contain hydroxy acids. Examples of the hydroxy acid include citric acid, malic acid and lactic acid. By containing such hydroxy acid, it is possible to suppress the segregation of the metal compound after drying.

As the impregnation method of impregnating the zirconia calcined body with the impregnating solution, any method can be used without particular limitation as long as the impregnating solution can infiltrate into the pores of the porous zirconia molded body. In a simple and preferable method, the whole and/or a part of the porous zirconia molded body is immersed in the impregnating solution. In this case, the impregnating solution can permeates by the capillarity into an inside of the porous zirconia molded body. The impregnating time may be 3 minutes or more, and may be 10 minutes to 10 hours.

The impregnating solution may be permeated in the whole of the porous zirconia molded body and may be permeated in an arbitrary portion of the porous zirconia molded body. The method of impregnating only an arbitrary portion is not particularly limited, and examples thereof include control of the weight and volume of the impregnating solution and control of the impregnation time.

A plurality of impregnating solutions may be used for one zirconia calcined body. In a specific example, after permeating the first type of impregnating solution from one end, the second type of impregnating solution is impregnated from the same end or the other end.

The atmosphere in impregnating the impregnating solution is not particularly limited, and an ambient air, an inert gas and the like can be used under normal pressure, reduced pressure, and pressurized conditions. Since no special facilities is required, it is preferable to perform under normal atmospheric pressure. From the viewpoint of shortening the preparation time, a reduced pressure atmosphere or a pressurized atmosphere is preferable as the surrounding environment because of improving the permeation of the impregnating liquid. Furthermore, it is effective in shortening the time required for the impregnation step to repeat multiple times a decompression operation and operation returning to normal pressure (decompression/normal pressure operation) or a pressurization operation and operation returning to normal pressure (pressurization/normal pressure operation). The time for immersing the zirconia calcined body in the impregnating solution is not universally determined depending on the size of the zirconia calcined body, the degree of penetration of the impregnating solution, the immersion method and the like and can be adjusted appropriately. For example, the time for immersing is usually 1 to 120 hours in the case of immersing, the time for immersing is usually 0.5 to 12 hours in the case of immersing under reduced pressure, and the time for immersing is usually 0.2 to 6 hours in the case of contacting under pressurization.

It is preferable to comprise a drying step after the impregnating step. The drying step is a step of drying the porous zirconia molded body in which the metal compound is deposited. It is preferable that the drying temperature is within a range of 50 to 200 °C, and the drying time is within a range of 15 minutes to 20 hours. It is preferable to sufficiently vaporize the solvent after drying. In some cases, the heat treatment step may be carried out following this drying step. The drying step may also be included in the heat treatment step. It is possible to comprise a degreasing step after the impregnating step. The degreasing step is a step of removing unnecessary components, such as organic substance contained in the impregnation solution. The degreasing temperature may be within a range of 200 to 600 °C, and the degreasing time is within a range of 50 hours to 200 hours. By degreasing, it is possible to completely remove unnecessary components. In some cases, the heat treatment step may be carried out following this degreasing step. The degreasing step may also be included in the heat treatment step.

Since the solvent, unreacted product, by-product and the like remain in the porous zirconia molded body in which the metal compound is deposited and supported, prepared in such a way, it is preferable to perform a heat treatment. The heat treatment method is not particularly limited, but heat treatment under normal atmospheric pressure is preferable since no special facilities is required. The heat treatment temperature is not particularly limited, but it is preferably within a range of 500 °C to 1200 °C. By heat treatment, impurities, organic substances and odors can be removed.

By such preparing method, a zirconia mill blank for dental cutting and machining of the present invention can be prepared. The prepared zirconia mill blank for dental cutting and machining is severed, cut, and polished so as to have a desired size as necessary.

The method for perfect sintering the zirconia mill blank for dental cutting and machining of the present invention is not particularly limited, but a simple and preferred method is to firing at normal pressure. The firing temperature is not particulary limited, but preferably 1450 to 1600 °C. The holding time at the firing temperature is not particularly limited, but is preferably 1 minute to 12 hours, and particularly preferably 2 to 4 hours. The temperature increase rate is not particularly limited, but is preferably 1 to 400 °C/min, and more preferably 3 to 100 °C/h.

The kind of a prosthesis device prepared by cutting and machining the zirconia mill blank for dental cutting and machining according to the present invention is not limited particularly, and there is no problem at all even if the prosthesis device is any of an inlay, an onlay, a veneer, a crown, a bridge and the like. Therefore, a shape of a zirconia mill blank for dental cutting and machining which is cut and machined for preparing a prosthesis device is not limited particularly, and any zirconia mill blank for dental cutting and machining can be used even if the zirconia mill blank for dental cutting and machining has any shape such as a block shape corresponding to an inlay, an onlay, a veneer, a crown and the like and a disk shape corresponding to a bridge.

### [Example]

The present invention is described in more detail and specifically with reference to Examples. However, the present invention is not limited to Examples.

### (Preparation of zirconia calcined body 1)

Zirconia powder containing 5.2 mol % of solid-solved yttria and not added with alumina in an amount of 486 g was filled in a mold (φ100 mm), and press molding (surface pressure: 50 MPa) was performed to obtain a molded body. Further, the molded body was subjected to CIP treatment (maximum load pressure: 200 MPa, holding period: 1 minute. Thereafter, calcination was performed in an electric furnace (1000 °C, 30 minutes) to prepare a zirconia calcined body having a diameter of 98.5 mm and a thickness of 18 mm.

### (Preparation of zirconia calcined body 2)

A zirconia calcined body was prepared in the same manner as the zirconia calcined body 1 expect that zirconia powder containing 5.2 mol % of solid solved yttria and 0.05 % by weight of alumina was used.

### (Preparation of zirconia calcined body 3)

A zirconia calcined body was prepared in the same manner as the zirconia calcined body 1 expect that zirconia powder containing 6.5 mol % of solid solved yttrium was used.

### (Preparation of zirconia calcined body 4)

A zirconia calcined body was prepared in the same manner as the zirconia calcined body 1 expect that zirconia powder prepared by ball mill mixing zirconia powder containing 5.2 mol % solid solved yttria and yttria powder added to achieve a total yttria concentration of 6.5 mol % and followed by spray drying was used.

### (Preparation of zirconia calcined body 5)

A zirconia calcined body was prepared in the same manner as the zirconia calcined body 1 expect that zirconia powder containing 3.0 mol % of solid solved yttrium was used.

### (Preparation of zirconia calcined body 6)

A zirconia calcined body was prepared in the same manner as the zirconia calcined body 1 expect that zirconia powder containing 4.0 mol % of solid solved yttrium was used.

### (Preparation of impregnating solution)

Table 1 shows the compositions of the impregnating solutions. According to the composition shown in Table 1, 100 g of the impregnating solution was prepared by adding a metal salt and an additive to ion-exchanged water and stirring for 1 hour.

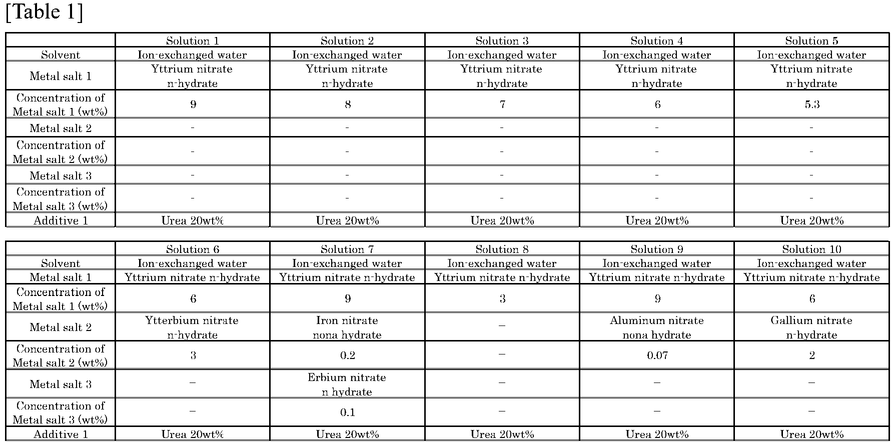

### (Impregnation of impregnating solution into zirconia calcined body)

A plastic container was placed on a horizontal workbench, and the zirconia calcined body was placed inside. The impregnating solution was poured so that the liquid level reached 80% of the thickness of the zirconia calcined body to leave to stand for 15 hours.

### (Heat treatment after impregnation)

When the impregnating solution contained urea, heat treatment was performed after impregnation of the impregnating solution. The zirconia calcined body after impregnation was placed in a resin bag and degassed. The porous zirconia molded body placed in a resin bag and degassed was placed in a dryer, and then a heat treatment at 95 °C for 15 hours was performed to deposit a metal compound. After the heat treatment, the porous zirconia molded body was taken out from the resin bag and dried (120 °C, 1 hour) to prepare a porous zirconia molded body supporting a metal compound.

### (Removal of residual organic matter)

The zirconia calcined body after impregnation and/or heat treatment was heat treated in an electric furnace (500 °C, 30 min) to remove residual organic matter to prepare a zirconia mill blank for dental cutting and machining in which a metal compound is supported in a pore.

### (Repeat of impregnation)

In Examples 6 and Example 13, the steps from the impregnating step to the removal of residual organic matter were repeated multiple times.

### (Partial impregnation)

In Example 11, the zirconia calcined body 1 was placed on a horizontal workbench. A jig was attached to the top surface of the zirconia calcined body 1 to retain the solution. Solution 1 was poured onto the top surface of the zirconia calcined body 1 at 30 g. The solution 1 was partially impregnated by allowing it to stand until the entire amount was absorbed by the zirconia calcined body.

### (Preparation of test specimen for stabilizing material measurement and for translucency measurement)

A test specimen having a diameter of 12 mm × thickness of 2.0 mm was prepared by using a dental milling machine (manufactured by Roland Corporation, DWX51D) from the central portion of the zirconia mill blank for dental cutting and machining in which metal compound was supported within the pores.

### (Evaluation of amount of stabilizer)

A molar fraction of stabilizer in terms of oxide (in the case of yttrium, the stabilizer oxide Y₂O₃) contained in each test specimen was measured by using a fluorescent X-ray apparatus (manufactured by Rigaku Corporation). Within the scope of Example and Comparative Example, the stabilizer concentration was calculated using yttrium and ytterbium as the stabilizer. Measurement was performed on both the top and bottom surfaces of each test specimen, and the average of these measurements was taken as the value for the test specimen.

### (Evaluation of translucency)

Each test specimen after evaluation of amount of stabilizer was perfect sintered (rate of temperature increase: 3°C/min, firing temperature: high temperature (1500 °C to 1600 °C) and low temperature (1400 °C to 1500 °C), holding time: 1 hours) in a firing furnace. Then, each test specimen was adjusted to have the thickness (1.0 mm) with a surface grinder. The translucency was evaluated using the total light transmittance at an irradiation wavelength of 700 nm from the transmission spectrum measured with an ultraviolet-visible spectrophotometer (V-750, manufactured by JASCO Corporation).

Translucency was scored based on the following criteria:
3: total light transmittance at 700 nm (high temparature sintering) ≥ 50 %, and total light transmittance at 700 nm (low temparature sintering) > 35 %
2: 50 % > total light transmittance at 700 nm (high temparature sintering) ≥ 44 %, and total light transmittance at 700 nm (low temparature sintering) > 35 %
1: 44 % > total light transmittance at 700 nm (high temparature sintering) ≥ 37 %, and total light transmittance at 700 nm low temparature sintering) > 35 %
0: 37 % > total light transmittance at 700 nm (high temparature sintering), or 35 % > total light transmittance at 700 nm (low temparature sintering)

Furthermore, the light transmittance difference between the high temparature sintered body and the low temparature sintered body was scored using the following criteria.
3: total light transmittance difference at 700 nm ≥ 6 % pt
2: 6 % pt > total light transmittance difference at 700 nm ≥ 3 % pt
1: 3 % pt > total light transmittance difference at 700 nm ≥ 0.5 % pt
0: 0.5 % pt > total light transmittance difference at 700 nm

### (Evaluation of crystal grain size)

The microstructure of each test specimen was observed using a scanning electron microscope (manufactured by JEOL Ltd.). The test specimen used for Evaluation of translucency was mirror-polished, was fired for 10 minutes at a temperature (1500 °C or 1400 °C) that is 50 °C lower than the sintering temperature (1550 °C or 1450 °C) as thermally etchin in order to facilitate observation of crystal grains that are difficult to observe due to polishing, and was vapor-deposited with gold on the surface to use as the test specimen. From the obtained microscopic images, the particle size distribution (Heywood diameter, volume distribution) of each specimen was measured using image analysis particle size distribution measurement software (Mac-View, manufactured by MOUNTECH Co., Ltd.), and the median diameter was taken as the crystal grain size of each specimen at the sintering temperature (1550 °C or 1450 °C).

### (Comprehensive evaluation)

Using the scores for evaluation of translucency and evaluation of light transmittance difference, a comprehensive evaluation was conducted based on the following criteria.
A: score for evaluation of translucency × score for evaluation of light transmittance difference ≥ 6
B: 6 > score for evaluation of translucency × score for evaluation of light transmittance difference ≥ 1 C: score for evaluation of translucency × score for evaluation of light transmittance difference = 0

When the comprehensive evaluation is A, both the translucency adjustment function based on sintering temperature change and the translucency are excellent, and at least one of them is particularly excellent, it is possible to suitable use for a wide range of applications requiring different translucency.

When the comprehensive evaluation is B, both the translucency adjustment function based on sintering temperature change and the translucency are excellent, it is possible to use for a wide range of applications requiring different translucency.

When the comprehensive evaluation is C, because at least one of translucency adjustment function based on sintering temperature change and translucency is insufficient, therefore, it can only be used for specific application.

Tables 2 and 3 show the preparation conditions and characteristic test results for zirconia mill blank for dental cutting and machining prepared in Examples and Comparative Examples.

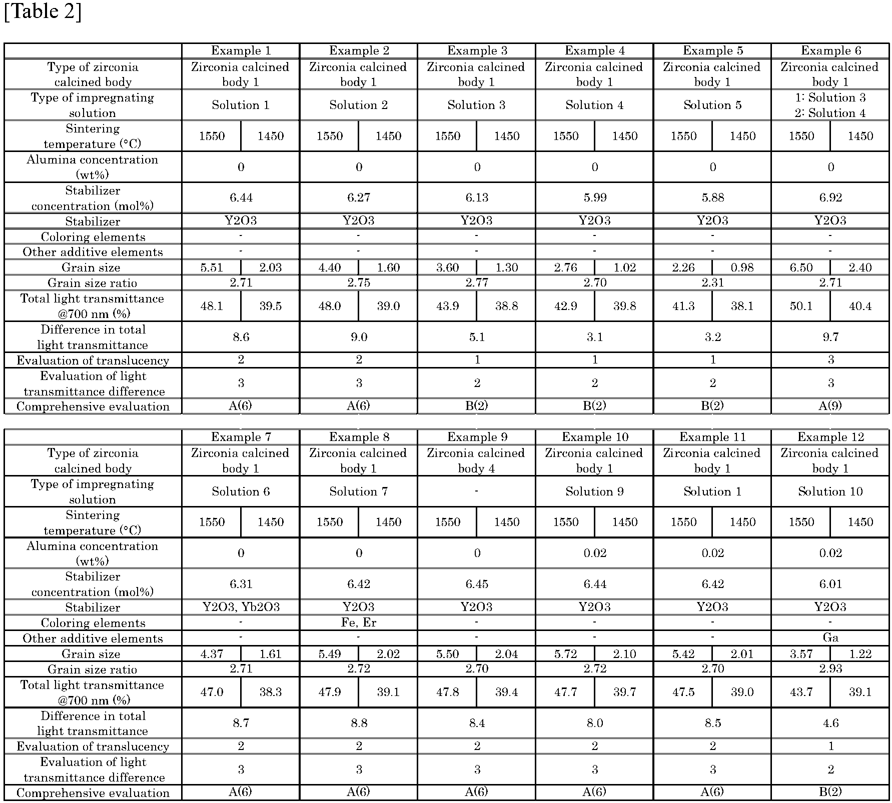

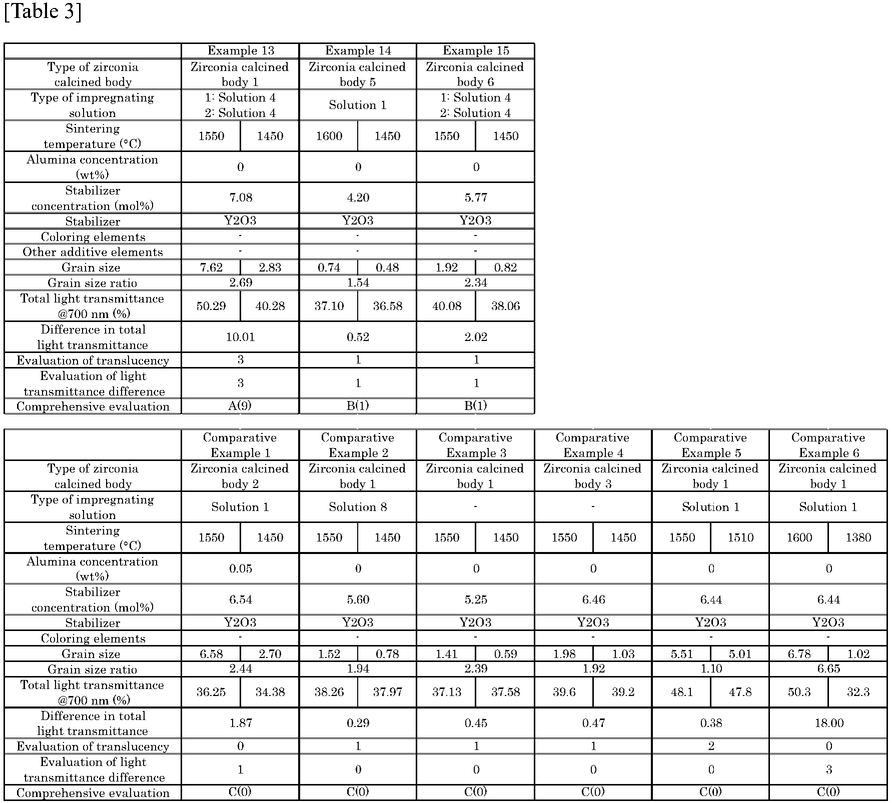

In Examples 1 to 15, it was confirmed that because of having both translucency adjustability by changing the sintering temperature and translucency, it was possible to use for multiple applications requiring different translucency.

In Comparative Example 1, it was confirmed that because the total light transmittance was low in both the high-temperature sintered body and the low-temperature sintered body was low and it was possible to use for only an application not requiring translucency.

In Comparative Examples 2 to 5, it was confirmed that the difference in total light transmittance between the high-temperature sintered body and the low-temperature sintered body was small and the translucency could not be changed significantly, and therefore it was not possible to use for multiple applications requiring different translucency.

In Comparative Example 6, it was confirmed that because the sintering temperature in the low-temperature sintering was too low, the translucency was low, and therefore it was not possible to use for multiple applications requiring different translucency in the case of obtaining a zirconia sintered body having excellent translucency.

With respect to the use of substantially any plural and/or singular terms herein, those having skill in the art can translate from the plural to the singular and/or from the singular to the plural as is appropriate to the context.

Although the description herein has been given with reference to the drawings and embodiments, it should be noted that those skilled in the art may make various changes and modifications on the basis of this invention without difficulty. Accordingly, any such changes and modifications are intended to be included in the scope of the embodiments.

### [Industrial applicability]

According to the present disclosure, it is possible to provide a zirconia mill blank for dental cutting and machining that can reduce the number of type of the zirconia mill blank for dental cutting and machining that dental laboratories and similar facilities must stock in order to obtain a zirconia sintered body with excellent translucency.

## Claims

1. A zirconia mill blank for dental cutting and machining for obtaining zirconia sintered bodies having different optical properties by different sintering temperatures, wherein
the different sintering temperatures are selected from at least a range of 1500 °C to 1600 °C and a range of 1400 °C to 1500 °C.

2. A zirconia mill blank for dental cutting and machining for obtaining zirconia sintered bodies having different optical properties by different sintering temperatures, wherein
the different sintering temperatures are in a range of 1400 °C to 1600 °C, and
a difference in sintering temperature bewtween the different sintering temperatures is 50 °C to 150°C.

3. A zirconia mill blank for dental cutting and machining for obtaining zirconia sintered bodies having different optical properties by different sintering temperatures, wherein
a difference between a total light transmittance of sintered body having a thickness of 1mm produced by sintering at one sintering temperature of the different sintering temperatures for 1 hour and a total light transmittance of sintered body having a thickness of 1mm produced by sintering at another sintering temperature of the different sintering temperatures for 1 hour is 0.5 % pt or more, and
a total light transmittance at light of wavelength of 700 nm in the case of sintering at a temperature in a range of 1500 °C to 1600 °C is 37 % or more.

4. A zirconia mill blank for dental cutting and machining for obtaining zirconia sintered bodies having different optical properties by different sintering temperatures, wherein
a crystal grain size of sintered body in the case of producing by sintering at one sintering temperature of the different sintering temperatures is 2.0 times or more of a crystal grain size of sintered body in the case of producing by sintering at another sintering temperature of the different sintering temperatures.
